# EUROPEAN PATENT APPLICATION

(11) **EP 1 099 684 A1**
(43) Date of publication of application: **16.05.2001**
(21) Application number: 00927772.4
(22) Date of filing: 16.05.2000
(51) Int. Cl.: C07C 201/06, C07C 205/02, C07C 205/05, C07C 205/06, C07B 61/00, B01D 53/34, B01D 53/56, B01D 53/77

(54) **PROCESS FOR THE PREPARATION OF NITRO COMPOUNDS AND METHOD FOR THE REMOVAL OF NITROGEN DIOXIDE**

(30) Priority: 17.05.1999 JP 13633999
(71) Applicant: Daicel Chemical Industries, Ltd., Osaka 590-8501 (JP)
(72) Inventor: ISHII, Yasutaka, Takatsuki-shi, Osaka 569-1112 (JP); NAKANO, Tatsuya, Himeji-shi, Hyogo 670-0029 (JP)
(74) Representative: Grünecker, Kinkeldey, Stockmair & Schwanhäusser Anwaltssozietät
(86) International application number: JP0003112
(87) International publication number: WO0069803

(57) **Abstract**

In the invented process for producing a nitro compound, an organic substrate and nitrogen dioxide are reacted in the presence of oxygen or are reacted in a molar ratio of nitrogen dioxide to the organic substrate of less than 1 to yield a corresponding nitro compound. The reaction may be performed in the presence of N-hydroxyphthalimide or other imide compounds. Such organic substrates include (a) aliphatic hydrocarbons, (b) alicyclic hydrocarbons, (c) non-aromatic heterocyclic compounds each having a carbon atom on a ring, which carbon atom is bonded to a hydrogen atom, (d) compounds each having a carbon-hydrogen bond at the adjacent position to an aromatic ring, and (e) compounds each having a carbon-hydrogen bond at the adjacent position to a carbonyl group. This process can efficiently nitrate an organic substrate even under relatively mild conditions.

## Description

### Technical Field

This invention relates to a process for producing a nitro compound, which is capable of directly and efficiently nitrating an organic substrate by using nitrogen dioxide, and to a process and system for eliminating nitrogen dioxide, which are capable of efficiently eliminating nitrogen dioxide from a nitrogen dioxide-containing gas through reaction.

### Background Art

Nitro compounds are in wide use as raw materials for pharmaceuticals, agricultural chemicals, dyestuffs, solvents, and explosives, and materials for amino compounds. To nitrate hydrocarbons, a nitric acid method using mixed acid (a mixture of nitric acid and sulfuric acid) is widely employed. However, the nitric acid method requires large amounts of a strong acid in a high concentration and the nitration reaction is an exothermic reaction. The ease of operation of the reaction cannot be therefore significantly improved. In the nitric acid method, large amounts of nitrogen oxides are by-produced which might contaminate the environment and such by-products must be removed through complicated treatments. In addition, the nitric acid method cannot yield corresponding nitro compounds from saturated hydrocarbons in high yields.

A process is known, which includes the step of reacting propane or another alkane with nitric acid in a gas phase to yield a nitro compound. The reaction according to this process, however, must be performed at such high temperatures of about 410°C and the selectivity of the reaction is low.

As a nitration process of an organic substrate, a process of nitrating an aromatic compound such as toluene or an alicyclic compound such as adamantane with N₂O₅ and ozone in the presence of an iron catalyst has been proposed. This process utilizes NO₃ as a reactant and can smoothly proceed nitration at low temperatures. However, the process requires extra utilities such as an ozonizer for generating ozone.

Separately, nitrogen dioxide and other NOₓ gases contained in exhaust gases from cars and factories cause air pollution, and techniques for eliminating these NOₓ gases have been proposed and performed. However, demands have been made to provide processes and facilities which are capable of more easily and efficiently eliminating NOₓ gases, from the viewpoint of recent higher requirements in environmental issues.

### Disclosure of Invention

Accordingly, an object of the invention is to provide a process for producing a nitro compound, which is capable of efficiently nitrating a substrate even under relatively mild conditions.

Another object of the invention is to provide a process for producing a nitro compound, which is capable of nitrating saturated hydrocarbons under mild conditions.

A further object of the invention is to provide a process and system for eliminating nitrogen dioxide, which are capable of efficiently eliminating nitrogen dioxide that will cause environmental pollution.

The present inventors made intensive investigations to achieve the objects, and found that a nitration reaction can efficiently proceed when an organic substrate is reacted with nitrogen dioxide under specific conditions. The invention has been accomplished based on these findings.

Specifically, the invention provides, in an aspect, a process for producing a nitro compound. The process includes the step of reacting nitrogen dioxide with an organic substrate in the presence of oxygen to yield a corresponding nitro compound.

In another aspect, the invention provides a process for producing a nitro compound. This process includes the step of reacting nitrogen dioxide with an organic substrate in a molar ratio of nitrogen dioxide to the organic substrate (nitrogen dioxide/organic substrate) of less than 1 to yield a corresponding nitro compound.

In the above processes, nitrogen dioxide may be reacted with the organic substrate in the presence of an imide compound of the following formula (1): 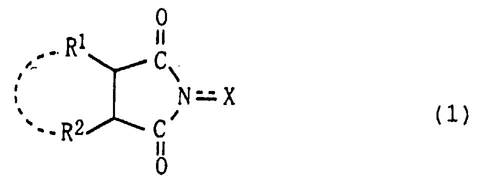 wherein each of R¹ and R² is, identical to or different from each other, a hydrogen atom, a halogen atom, an alkyl group, an aryl group, a cycloalkyl group, a hydroxyl group, an alkoxy group, a carboxyl group, an alkoxycarbonyl group, or an acyl group, where R¹ and R² may be combined to form a double bond, or an aromatic or non-aromatic ring; X is an oxygen atom or a hydroxyl group, where one or two N-substituted cyclic imido groups indicated in the formula (1) may be further formed on the substituent R¹ or R², or on the double bond or aromatic or non-aromatic ring formed together by R¹ and R².

The organic substrate may be selected from (a) aliphatic hydrocarbons, (b) alicyclic hydrocarbons, (c) non-aromatic heterocyclic compounds each having a carbon atom on a ring, which carbon atom is bonded to a hydrogen atom, (d) compounds each having a carbon-hydrogen bond at the adjacent position to an aromatic ring, and (e) compounds each having a carbon-hydrogen bond at the adjacent position to a carbonyl group.

In a further aspect, the invention provides a process for eliminating nitrogen dioxide. The process includes the step of bringing a gas containing nitrogen dioxide into contact with an organic substrate in the presence of oxygen to eliminate the nitrogen dioxide through reaction.

The invention provides, in yet another aspect, a process for eliminating nitrogen dioxide. This process includes the step of bringing a gas containing nitrogen dioxide into contact with an organic substrate in a proportion exceeding 1 mole relative to 1 mole of the nitrogen dioxide to thereby eliminate the nitrogen dioxide through reaction.

In the processes for eliminating nitrogen dioxide, the gas containing nitrogen dioxide may be brought into contact with the organic substrate in the presence of the imide compound of the formula (1).

As the organic substrate, at least one compound selected from the above compounds (a) to (e) may be used.

In another aspect, the invention provides a system for eliminating nitrogen dioxide. The system includes an eliminating means which brings a gas containing nitrogen dioxide into contact with an organic substrate in the presence of oxygen to eliminate the nitrogen dioxide through reaction.

In addition and advantageously, the invention provides a system for eliminating nitrogen dioxide. The system includes an eliminating means which brings a gas containing nitrogen dioxide into contact with an organic substrate in a proportion exceeding 1 mole relative to 1 mole of the nitrogen dioxide to thereby eliminate the nitrogen dioxide through reaction. Best Mode for Carrying Out the Invention

### [Organic Substrate]

Organic substrates for use in the invention include, but are not limited to, a wide variety of saturated and unsaturated compounds such as hydrocarbons (aliphatic hydrocarbons, alicyclic hydrocarbons, and aromatic hydrocarbons), heterocyclic compounds, alcohols, ethers, esters, ketones, and aldehydes. Each of these organic substrates can be used alone or in combination.

Preferred organic substrates include, for example, compounds each having a non-aromatic group with a carbon-hydrogen bond. Such compounds include, for example, (a) aliphatic hydrocarbons, (b) alicyclic hydrocarbons, (c) non-aromatic heterocyclic compounds each having a carbon atom on a ring, which carbon atom is bonded to a hydrogen atom, (d) compounds each having a carbon-hydrogen bond at the adjacent position to an aromatic ring, and (e) compounds each having a carbon-hydrogen bond at the adjacent position to a carbonyl group.

The aliphatic hydrocarbons (a) include, for example, (a1) alkanes, and (a2) alkenes and alkynes each having a carbon-hydrogen bond at the adjacent position to an unsaturated bond. Illustrative alkanes (a1) are methane, ethane, propane, butane, 2-methylpropane, pentane, 2-methylbutane, hexane, 2,3-dimethylbutane, 2-methylpentane, 3-methylpentane, heptane, 2-methylhexane, 3-methylhexane, 2,3-dimethylpentane, 2,4-dimethylpentane, 2-ethylpentane, 3-ethylpentane, octane, 2-methylheptane, 4-methylheptane, 2-ethylhexane, 2,3-dimethylhexane, 2,5-dimethylhexane, 2-propylhexane, 2,3,4-trimethylpentane, nonane, 2-methyloctane, 3-methyloctane, 2,6-dimethylheptane, 2-ethylheptane, 3-ethylheptane, decane, 2-methylnonane, 2,7-dimethyloctane, undecane, dodecane, tetradecane, hexadecane, octadecane, icosane, triacontane, and other straight- or branched-chain alkanes each having about 1 to 30 carbon atoms. Preferred alkanes include straight- or branched-chain alkanes each having about 1 to 20 carbon atoms, especially about 3 to 14 carbon atoms.

Examples of (a2) alkenes and alkynes each having a carbon-hydrogen bond at the adjacent position to an unsaturated bond are propylene, 1-butene, 2-butene, butadiene, 1-pentene, 2-pentene, isoprene, 1-hexene, 2-hexene, 1,5-hexadiene, 2,3-dimethyl-2-butene, 3-hexene, 1-heptene, 2-heptene, 1,6-heptadiene, 1-octene, 2-octene, 3-octene, 1,7-octadiene, 2,6-octadiene, 2-methyl-2-butene, 1-nonene, 2-nonene, decene, decadiene, dodecene, dodecadiene, dodecatriene, undecene, undecadiene, undecatriene, and other straight- or branched-chain alkenes and alkanes each having about 3 to 30 carbon atoms, and preferably about 3 to 12 carbon atoms.

The alicyclic hydrocarbons (b) include, for example, (b1) cycloalkanes, (b2) cycloalkenes, and (b3) bridged cyclic hydrocarbons.

Such cycloalkanes (b1) include, but are not limited to, cyclopropane, cyclobutane, cyclopentane, methylcyclopentane, cyclohexane, methylcyclohexane, 1,2-dimethylcyclohexane, ethylcyclohexane, isopropylcyclohexane, cycloheptane, cyclooctane, methylcyclooctane, cyclononane, cyclodecane, cyclododecane, cyclotridecane, cyclotetradecane, cyclopentadecane, cyclohexadecane, cyclooctadecane, cyclononadecane, and other cycloalkanes each having about 3 to 30 carbon atoms, and preferably about 3 to 20 carbon atoms.

Illustrative cycloalkenes (b2) are cyclopropene, cyclobutene, cyclopentene, cyclohexene, 1-methyl-1-cyclohexene, cycloheptene, cyclooctene, cyclononene, cyclodecene, cyclododecene, and other C₃-C₃₀ cycloalkenes; cyclopentadiene, cyclohexadiene, cycloheptadiene, cyclooctadiene, cyclodecadiene, cyclododecadiene, and other C₅-C₃₀ cycloalkadienes; cycloalkatrienes; cycloalkatetraenes; dihydronaphthalene, indene, fluorene, indan, tetralin, acenaphthene, phenalene, indanone, xanthene, and other condensed polycyclic hydrocarbons each having a 5- to 8-membered non-aromatic ring condensed to an aromatic ring.

The bridged cyclic hydrocarbons (b3) include, but are not limited to, decalin, bicyclo[2.2.0]hexane, bicyclo[2.2.2]octane, bicyclo[3.2.1]octane, bicyclo[4.3.2]undecane, thujone, carane, pinane, pinene, bornane, bornylene, norbornane, norbornene, camphor, camphoric acid, camphene, tricyclene, tricyclo[4.3.1.1^{2,5}]undecane, tricyclo[5.2.1.0^{3,8}]decane, exotricyclo[5.2.1.0^{2,6}]decane, endotricyclo[5.2.1.0^{2,6}]decane, endotricyclo[5.2.2.0^{2,6}]undecane, adamantane, 1-adamantanol, 1-chloroadamantane, 1-methyladamantane, 1,3-dimethyladamantane, 1-methoxyadamantane, 1-carboxyadamantane, 1-methoxycarbonyladamantane, 1-nitroadamantane, tetracyclo[4.4.0.1^{2,5}.1^{7,10}]dodecane, perhydroanthracene, perhydroacenaphthene, perhydrophenanthrene, perhydrophenalene, perhydroindene, and derivatives of these compounds.

Illustrative non-aromatic heterocyclic compounds (c) each having a carbon atom on a ring, which carbon atom is bonded to a hydrogen atom, include 5- or 6-membered heterocyclic compounds having a hetero atom selected from a nitrogen atom, an oxygen atom and a sulfur atom, and condensed heterocyclic compounds each having a 5- or 6-membered heterocyclic ring having a hetero atom bonded to an aromatic ring. Such heterocyclic rings include, but are not limited to, dihydrofuran, tetrahydrofuran, pyran, dihydropyran, tetrahydropyran, pyrrolidine, piperidine, piperazine, morpholine, indoline, chroman, and isochroman.

The compounds (d) each having a carbon-hydrogen bond at the adjacent position to an aromatic ring include, for example, (d1) aromatic hydrocarbons each having an alkyl group on an aromatic carbocyclic ring, such as toluene, xylene, 1-ethyl-4-methylbenzene, 1-ethyl-3-methylbenzene, 1-t-butyl-4-methylbenzene, 1-methoxy-4-methylbenzene, mesitylene, durene, methylnaphthalene, methylanthracene, 4,4'-dimethylbiphenyl, ethylbenzene, propylbenzene, 1,4-diethylbenzene, and diphenylmethane; and (d2) heterocyclic compounds each having an alkyl group on an aromatic heterocyclic ring, such as 2-methylfuran, 3-methylfuran, 3-methylthiophene, 2-methylpyridine, 3-methylpyridine, 4-methylpyridine, 2,4-dimethylpyridine, 2,4,6-trimethylpyridine, 4-methylindole, 2-methylquinoline, 2-ethylfuran, 3-propylthiophene, 4-ethylpyridine, and 4-butylquinoline.

Illustrative compounds (e) each having a carbon-hydrogen bond at the adjacent position to a carbonyl group are aldehydes, ketones, carboxylic acids and derivatives thereof.

Such aldehydes include, for example, aliphatic aldehydes such as acetaldehyde, propionaldehyde, butyraldehyde, isobutyraldehyde, pentyl aldehyde, hexyl aldehyde, heptyl aldehyde, octyl aldehyde, nonyl aldehyde, decyl aldehyde, malonaldehyde, succinaldehyde, adipaldehyde, and sebacaldehyde; and alicyclic aldehydes such as formylcyclohexane and cycloneral.

The ketones include aliphatic ketones, cyclic ketones, aromatic ketones, and heterocyclic ketones. Illustrative aliphatic ketones include acetone, methyl ethyl ketone, methyl isopropyl ketone, methyl isobutyl ketone, methyl t-butyl ketone, 2-pentanone, 3-pentanone, 2-hexanone, 3-hexanone, 2-heptanone, 3-heptanone, 4-heptanone, 2-octanone, 3-octanone, 4-octanone, 2-nonanone, and 2-decanone. Illustrative cyclic ketones are cyclopentanone, cyclohexanone, methylcyclohexanone, dimethylcyclohexanone, cycloheptanone, isophorone, cycloheptanone, cyclooctanone, cyclononanone, cyclodecanone, cyclohexadione, cyclooctadione, and other non-aromatic cyclic mono- or poly-ketones; α-tetralone, β-tetralone, indanone, and other cyclic ketones each having an aromatic ring; 2-adamantanone, 1-methyl-2-adamantanone, 1,3-dimethyl-6-adamantanone, menthone, and other bridged cyclic ketones. Illustrative aromatic ketones are acetophenone, and propiophenone; and illustrative heterocyclic ketones include acetylpyridine.

Examples of the carboxylic acids and derivatives thereof include aliphatic dicarboxylic acids and derivatives thereof, such as malonic acid and its esters, succinic acid and its esters, and glutaric acid and its esters.

Each of these organic substrates may have a substituent. Such substituents include, but are not limited to, halogen atoms, oxo group, hydroxyl group, mercapto group, substituted oxy groups (e.g., alkoxy groups, aryloxy group, and acyloxy groups), substituted thio groups, carboxyl group, substituted oxycarbonyl group, substituted or unsubstituted carbamoyl groups, cyano group, nitro group, substituted or unsubstituted amino groups, alkyl groups, alkenyl group, alkynyl groups, cycloalkyl groups, cycloalkenyl groups, aryl groups (e.g., phenyl and naphthyl groups), aralkyl groups, and heterocyclic groups.

### [Nitrogen Dioxide and Oxygen]

The nitrogen dioxide (NO₂) may be a pure nitrogen dioxide or a mixture containing other components. Nitrogen dioxide formed in a reaction system can also be employed.

The proportion of the nitrogen dioxide may be equivalent mole, or excess moles (e.g., 1 to 5 times by mole) relative to the organic substrate, or less than the equivalent mole relative to the organic substrate. The use of nitrogen dioxide in a proportion relative to 1 mole of the organic substrate of less than 4 moles, preferably less than 1 mole (e.g., 0.0001 mole or more and less than 1 mole), more preferably 0.8 mole or less (e.g., 0.001 to 0.8 mole), typically preferably 0.5 mole or less (e.g., 0.002 to 0.5 mole), and particularly 0.25 mole or less (e.g., 0.005 to 0.25 mole) markedly improves the conversion rate of nitrogen dioxide and the selectivity of the reaction. For example, when toluene or another compound having a carbon-hydrogen bond at the adjacent position (α-position) to an aromatic ring is used as the substrate and the molar ratio of nitrogen dioxide to the organic substrate (nitrogen dioxide/organic substrate) is large, large amounts of compounds having a nitro group introduced into the aromatic ring are by-produced, in addition to a compound having a nitro group introduced into the α-position of the aromatic ring. However, by setting the molar ratio at less than 4, particularly less than 1, nitration on the α-position of the aromatic ring selectively proceeds.

When the molar ratio is set at less than 1, the conversion rate of nitrogen dioxide is markedly increased. Particularly, when nitrogen dioxide is to be eliminated from a gas containing nitrogen dioxide, more than 1 mole of the organic substrate relative to 1 mole of nitrogen dioxide is preferably used.

If the molar ratio is set at less than 1 mole, a high conversion rate of nitrogen dioxide and a high reaction selectivity can be obtained even in the absence of oxygen.

A reaction of the organic substrate with nitrogen dioxide in the presence of oxygen markedly increases the reaction rate. This advantage is outstanding when aliphatic hydrocarbons and/or alicyclic hydrocarbons are employed as the organic substrate.

The oxygen for use in the invention may be a pure oxygen or a diluted oxygen diluted with an inert gas such as carbon dioxide, nitrogen, helium, or argon gas. Air can also be used as the oxygen source. The amount of oxygen is 0.5 mole or more (e.g., 1 mole or more), preferably about 1 to 100 moles, and more preferably about 2 to 50 moles relative to 1 mole of the nitrogen dioxide. Excess moles of oxygen is often used relative to the nitrogen dioxide.

### [Imide Compound]

A reaction of the organic substrate with nitrogen dioxide in the presence of the imide compound of the formula (1) markedly increases the reaction rate of nitration. This advantage is outstanding when aliphatic hydrocarbons and/or alicyclic hydrocarbons are employed as the organic substrate. When toluene or another compound having a carbon-hydrogen bond at the adjacent position (α-position) to an aromatic ring is used as the substrate, nitration on the aromatic ring preferentially proceeds in the absence of the imide compound, but nitration on the α-position of the aromatic ring highly selectively proceeds in the presence of the imide compound.

Of the substituents R¹ and R² in the imide compound of the formula (1), the halogen atom includes iodine, bromine, chlorine and fluorine atoms. The alkyl group includes, for example, methyl, ethyl, propyl, isopropyl, butyl, isobutyl, s-butyl, t-butyl, pentyl, hexyl, heptyl, octyl, decyl, and other straight- or branched-chain alkyl groups each having about 1 to 10 carbon atoms. Preferred alkyl groups are alkyl groups each having about 1 to 6 carbon atoms, and more preferably lower alkyl groups each having about 1 to 4 carbon atoms.

The aryl group includes phenyl and naphthyl groups, for example; and illustrative cycloalkyl groups include cyclopentyl and cyclohexyl groups. Illustrative alkoxy groups are methoxy, ethoxy, propoxy, isopropoxy, butoxy, isobutoxy, t-butoxy, pentyloxy, hexyloxy, and other alkoxy groups each having about 1 to 10 carbon atoms, preferably about 1 to 6 carbon atoms, of which lower alkoxy groups each having about 1 to 4 carbon atoms are especially preferred.

Examples of the alkoxycarbonyl group include methoxycarbonyl, ethoxycarbonyl, propoxycarbonyl, isopropoxycarbonyl, butoxycarbonyl, isobutoxycarbonyl, t-butoxycarbonyl, pentyloxycarbonyl, hexyloxycarbonyl, and other alkoxycarbonyl groups each having about 1 to 10 carbon atoms in the alkoxy moiety. Preferred alkoxycarbonyl groups are alkoxycarbonyl groups each having about 1 to 6 carbon atoms in the alkoxy moiety, of which lower alkoxycarbonyl groups each having about 1 to 4 carbon atoms in the alkoxy moiety are typically preferred.

The illustrative acyl groups include, but are not limited to, formyl, acetyl, propionyl, butyryl, isobutyryl, valeryl, isovaleryl, pivaloyl, and other acyl groups each having about 1 to 6 carbon atoms.

The substituents R¹ and R² may be identical to, or different from each other. The substituents R¹ and R² in the formula (1) may be combined to form a double bond, or an aromatic or non-aromatic ring. The preferred aromatic or non-aromatic ring is a 5- to 12-membered ring, and especially a 6- to 10-membered ring. The ring may be a heterocyclic ring or a condensed heterocyclic ring, but it is often a hydrocarbon ring. Such rings include, for example, non-aromatic alicyclic rings (e.g., cyclohexane ring and other cycloalkane rings which may have a substituent, cyclohexene ring and other cycloalkene rings which may have a substituent), non-aromatic bridged rings (e.g., 5-norbornene ring and other bridged hydrocarbon rings which may have a substituent), benzene ring, naphthalene ring and other aromatic rings (including condensed rings) which may have a substituent. The ring is composed of an aromatic ring in many cases. The ring may have a substituent such as an alkyl group, a haloalkyl group, a hydroxyl group, an alkoxy group, a carboxyl group, an alkoxycarbonyl group, an acyl group, a nitro group, a cyano group, an amino group, or a halogen atom.

In the formula (1), X represents an oxygen atom or a hydroxyl group, and the bond between the nitrogen atom N and X is a single bond or a double bond.

On the substituent R¹ or R², or on the double bond or aromatic or non-aromatic ring formed together by R¹ and R², one or two N-substituted cyclic imido groups indicated in the formula (1) may be further formed. For example, when R¹ or R² is an alkyl group having two or more carbon atoms, the N-substituted cyclic imido group may be formed together with adjacent two carbon atoms constituting the alkyl group. Likewise, when R¹ and R² are combined to form a double bond, the N-substituted cyclic imido group may be formed together with the double bond. In case that R¹ and R² are combined to form an aromatic or non-aromatic ring, the N-substituted cyclic imido group may be formed with adjacent two carbon atoms constituting the aforementioned ring.

Preferred imide compounds include compounds of the following formulae: 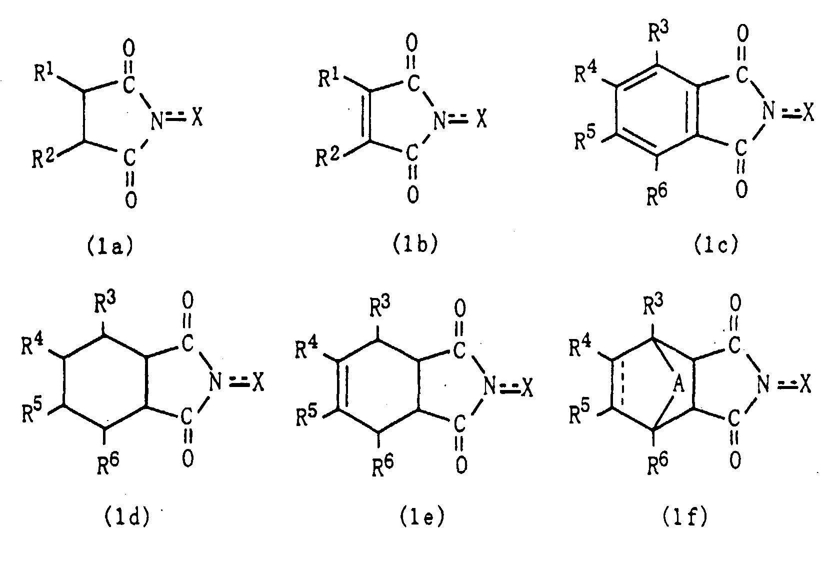 wherein R³ to R⁶ are each, identical to or different from one another, a hydrogen atom, an alkyl group, a haloalkyl group, a hydroxyl group, an alkoxy group, a carboxyl group, an alkoxycarbonyl group, an acyl group, a nitro group, a cyano group, an amino group, or a halogen atom, where adjacent groups of R³ to R⁶ may be combined with each other to form an aromatic or non-aromatic ring; in the formula (1f), A represents a methylene group or an oxygen atom, and R¹ and R² have the same meanings as defined above; and one or two N-substituted cyclic imido groups indicated in the formula (1c) may be further formed on the benzene ring in the formula (1c).

In the substituents R³ to R⁶, the alkyl group includes similar alkyl groups to those exemplified above, especially alkyl groups each having about 1 to 6 carbon atoms. The haloalkyl group includes trifluoromethyl group and other haloalkyl groups each having about 1 to 4 carbon atoms, and the alkoxy group includes similar alkoxy groups to those mentioned above, and especially lower alkoxy groups each having about 1 to 4 carbon atoms. The alkoxycarbonyl group includes similar alkoxycarbonyl groups to those described above, particularly lower alkoxycarbonyl groups each having about 1 to 4 carbon atoms in the alkoxy moiety. As the acyl group, there may be mentioned similar acyl groups to those described above, especially acyl groups each having about 1 to 6 carbon atoms. Illustrative halogen atoms include fluorine, chlorine and bromine atoms. Each of the substituents R³ to R⁶ is often a hydrogen atom, a lower alkyl group having about 1 to 4 carbon atoms, a carboxyl group, a nitro group, or a halogen atom. The ring formed together by R³ to R⁶ includes similar rings to the aforementioned rings which are formed together by R¹ and R². Among them, aromatic or non-aromatic 5- to 12-membered rings are particularly preferred.

Illustrative preferred imide compounds include N-hydroxysuccinimide, N-hydroxymaleimide, N-hydroxyhexahydrophthalimide, N,N'-dihydroxycyclohexanetetracarboximide, N-hydroxyphthalimide, N-hydroxytetrabromophthalimide, N-hydroxytetrachlorophthalimide, N-hydroxychlorendimide, N-hydroxyhimimide, N-hydroxytrimellitimide, N,N'-dihydroxypyromellitimide, and N,N'-dihydroxynaphthalenetetracarboximide.

The imide compounds of the formula (1) can be prepared by a conventional imidation process (a process for the formation of an imide). Such imidation processes includes a process which includes the steps of allowing a corresponding acid anhydride to react with hydroxylamine NH₂OH for ring-opening of an acid anhydride group, and closing the ring to form an imide.

Such acid anhydrides include succinic anhydride, maleic anhydride, and other saturated or unsaturated aliphatic dicarboxylic anhydrides, tetrahydrophthalic anhydride, hexahydrophthalic anhydride (1,2-cyclohexanedicarboxylic anhydride), 1,2,3,4-cyclohexanetetracarboxylic 1,2-dianhydride, and other saturated or unsaturated non-aromatic cyclic polycarboxylic anhydrides (alicyclic polycarboxylic anhydrides), HET anhydride (chlorendic anhydride), himic anhydride, and other bridged cyclic polycarboxylic anhydrides (alicyclic polycarboxylic anhydrides), phthalic anhydride, tetrabromophthalic anhydride, tetrachlorophthalic anhydride nitrophthalic anhydride, trimellitic anhydride, methylcyclohexenetricarboxylic anhydride, pyromellitic anhydride, mellitic anhydride, 1,8;4,5-naphthalenetetracarboxylic dianhydride, and other aromatic polycarboxylic anhydrides.

Typically preferred imide compounds include N-hydroxyimide compounds derived from alicyclic polycarboxylic anhydrides or aromatic polycarboxylic anhydrides, of which N-hydroxyphthalimide and other N-hydroxyimide compounds derived from aromatic polycarboxylic anhydrides are especially preferred.

Each of the imide compounds of the formula (1) can be used alone or in combination. The imide compound can be used as being supported by a carrier or support. As such carriers, activated carbon, zeolite, silica, silica-alumina, bentonite, and other porous carries are often employed. The proportion of the imide compound to the carrier is, for example, about 0.1 to 50 parts by weight, preferably about 0.5 to 30 parts by weight, and more preferably about 1 to 20 parts by weight, relative to 100 parts by weight of the carrier.

The amount of the imide compound can be selected in a wide range and is generally about 0.0001 to 1 mole, preferably about 0.001 to 0.5 mole, more preferably about 0.01 to 0.4 mole, and frequently about 0.05 to 0.35 mole, relative to 1 mole of either the organic substrate or the nitrogen dioxide that is used in a less amount.

### [Nitration Reaction]

A nitration reaction can be performed in the presence of or in the absence of a solvent. Such solvents include, but are not limited to, benzene and other aromatic hydrocarbons; dichloromethane, chloroform, 1,2-dichloroethane, dichlorobenzene, and other halogenated hydrocarbons; t-butanol, t-amyl alcohol, and other alcohols; acetonitrile, benzonitrile, and other nitriles; acetic acid, propionic acid, and other organic acids; formamide, acetamide, dimethylformamide (DMF), dimethylacetamide, and other amides; and mixtures of these solvents. Excess amounts of the organic substrate to nitrogen dioxide can be used as the solvent. Such use of the organic substrate in excess amounts to nitrogen dioxide can markedly improve the conversion rate of nitrogen dioxide and the selectivity of the reaction.

The reaction temperature can be selected according to, for example, the type of the substrate, and is usually about 0°C to 150°C, preferably about 25°C to 125°C, and more preferably about 30°C to 100°C. The reaction can be performed either at atmospheric pressures or under pressure, in any of a batch system, a semi-batch system, a continuous system, or another conventional system.

After the completion of the reaction, reaction products can be easily separated and purified by a conventional separation and purification means. Such separation and purification means include, for example, filtration, concentration, distillation, extraction, crystallization, recrystallization, adsorption, column chromatography, and other separation means, and combinations of these separation means.

According to the invention, the nitration reaction smoothly proceeds under mild conditions. For example, when a compound having a non-aromatic group with a carbon-hydrogen bond is used as the organic substrate, a carbon atom constituting the carbon-hydrogen bond is nitrated.

Particularly, the invention can achieve smooth nitration of not only compounds with an active hydrogen, such as compounds having a carbon-hydrogen bond at the adjacent position to an aromatic ring or carbonyl group, but also hexane and other aliphatic hydrocarbons, and cyclohexane and other alicyclic hydrocarbons. This significantly distinguishes the invention from conventional equivalents.

The degrees of ease of introducing nitro group are methine carbon atom > methylene carbon atom > methyl carbon atom. When a compound (e.g., toluene) having a methyl group at the adjacent position (so-called benzyl position) to an aromatic ring is used as the substrate, a nitro group is introduced into a carbon atom of the methyl group. Depending on conditions, however, a corresponding aromatic aldehyde (e.g., benzaldehyde) in which the methyl group is converted into a formyl group, or a compound with a nitro group introduced into the aromatic ring is formed. When a compound (e.g., ethylbenzene) having a methylene group at the adjacent position to an aromatic ring is employed as the substrate, a corresponding nitro compound (e.g., α-nitroethylbenzene), in which the methylene group is nitrated, is formed, and under some reaction conditions, an oxime compound (e.g., acetophenone oxime), in which the methylene group is converted into an oxime moiety, is formed.

The invention has features in that (i) by existing oxygen, the reaction rate can be markedly increased, and (ii) by using excess amounts of the organic substrate to nitrogen dioxide, the nitrogen dioxide can be highly efficiently converted and the reaction selectivity can be markedly improved. Accordingly, the use of the above nitration reactions can efficiently eliminate nitrogen dioxide from a nitrogen dioxide-containing gas.

Such nitrogen dioxide-containing gases for use in the invented process and system for eliminating nitrogen dioxide are not limited as far as gases contain nitrogen dioxide. For example, exhaust gases of factories, and exhaust gases of cars can be employed. As the organic substrate, any of the above-exemplified compounds can be employed, but compounds which are low-cost, have a high reactivity, and are easy to be treated in an aftertreatment are preferred. Such compounds include alkanes and other aliphatic hydrocarbons, and cycloalkanes and other alicyclic hydrocarbons. Each of the organic substrates may be used alone or in combination.

The means for eliminating nitrogen dioxide in the invented system can be composed of, for example, a vessel containing a mixture of the organic substrate and, where necessary, the imide compound of the formula (1), a nitrogen dioxide-containing gas inlet line for introducing the nitrogen dioxide-containing gas into the vessel, and where necessary, an oxygen supply line for supplying oxygen, and an exhaust gas line for discharging an exhaust gas from the vessel. The imide compound can be supported on a carrier, as mentioned above. The organic substrate may be gaseous. The elimination of nitrogen dioxide can be performed in any of a batch system, a semi-batch system and a continuous system.

The concentration of the imide compound in the mixture can be selected within an appropriate range in consideration of the efficiency of eliminating nitrogen dioxide, and is for example about 0.001 to 2 mole/L, preferably about 0.01 to 1 mole/L, and more preferably about 0.05 to 0.3 mole/L. The mixture may contain an inert solvent. The proportion of oxygen to be supplied is, for example, equal to or more than 1 time by mole that of the nitrogen dioxide. The temperatures and pressures for the contact of the nitrogen dioxide-containing gas with the organic substrate are the same as mentioned above.

According to the invented process and system for eliminating nitrogen dioxide, nitrogen dioxide in the nitrogen dioxide-containing gas can be smoothly reacted with the organic substrate under mild conditions and the nitrogen dioxide can be efficiently eliminated from the gas.

The product nitro compounds obtained by the invented production processes can be used as raw materials for pharmaceuticals, agricultural chemicals, dyestuffs, solvents, and explosives, and materials for amino compounds. For example, a lactam compound can be obtained by nitrating an alicyclic hydrocarbon to yield a nitro compound, reducing the nitro compound in a conventional manner to yield a corresponding oxime compound, and subjecting the oxime compound to Beckmann rearrangement to yield a corresponding lactam compound.

Product compounds formed through the reaction of the organic substrate with nitrogen dioxide in the invented process for eliminating nitrogen dioxide can be used as raw materials for the pharmaceuticals and the like. Alternatively, some product compounds may be subjected to, where necessary, reduction or another treatment, and to a conventional waste treatment such as activated sludge treatment.

### Industrial Applicability

The invented processes for producing a nitro compound reacts an organic substrate with nitrogen dioxide under specific conditions and can directly and efficiently nitrate the substrate even under relatively mild conditions to yield a corresponding nitro compound in a high yield. The invented processes can easily nitrate even alkanes and other aliphatic hydrocarbons or cycloalkanes and other alicyclic hydrocarbons under mild conditions.

The invented process and system for eliminating nitrogen dioxide allow nitrogen dioxide in a nitrogen dioxide-containing gas to smoothly react with an organic substrate under mild conditions and can efficiently eliminate the nitrogen dioxide from the gas. The process and system are markedly useful for inhibiting environmental pollution by nitrogen dioxide.

### Examples

The present invention will now be illustrated in more detail with reference to several examples below, which are not intended limiting the scope of the invention.

### EXAMPLE 1

In a flask, 5 ml (46.3 mmol) of cyclohexane, 0.6 mmol of N-hydroxyphthalimide, and 0.1 ml (3.24 mmol) of nitrogen dioxide were placed and the resulting mixture was reacted at 70°C in an air atmosphere (1 atm) for 14 hours with stirring. A gas chromatographic analysis of reaction products revealed that nitrocyclohexane and cyclohexyl nitrate were formed in yields of 75% and 11%, respectively on the basis of nitrogen dioxide.

### EXAMPLE 2

In a flask, 3 mmol of cyclohexane, 0.3 mmol of N-hydroxyphthalimide, 15 mmol of nitrogen dioxide, and 5 ml of trifluoromethylbenzene were placed, and the resulting mixture was reacted at 60°C in an oxygen atmosphere (1 atm) for 5 hours with stirring. A gas chromatographic analysis of reaction products revealed that nitrocyclohexane and cyclohexanol were formed in yields of 4.6% and 0.42%, respectively on the basis of cyclohexane. The conversion rate from cyclohexane was 40%.

### EXAMPLE 3

In a flask, 3.2 mmol of cyclododecane, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitrocyclododecane and cyclododecanone were formed in yields of 34% and 11%, respectively on the basis of cyclododecane.

### EXAMPLE 4

In a flask, 5 ml (41.3 mmol) of cycloheptane, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitrocycloheptane and cycloheptyl nitrate were formed in yields of 88% and 4%, respectively on the basis of nitrogen dioxide.

### EXAMPLE 5

In a flask, 5 ml (41.3 mmol) of cycloheptane and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitrocycloheptane and cycloheptyl nitrate were formed in yields of 50% and 13%, respectively on the basis of nitrogen dioxide.

### EXAMPLE 6

In a flask, 5 ml (35.2 mmol) of methylcyclohexane, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that 1-methyl-1-nitrocyclohexane and 1-methylcyclohexyl nitrate were formed in yields of 68% and 5%, respectively on the basis of nitrogen dioxide.

### EXAMPLE 7

In a flask, 5 ml (35.2 mmol) of methylcyclohexane and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that 1-methyl-1-nitrocyclohexane and 1-methylcyclohexyl nitrate were formed in yields of 17% and 5%, respectively on the basis of nitrogen dioxide.

### EXAMPLE 8

In a flask, 1 mmol of adamantane, 15 mmol of nitrogen dioxide, 0.1 mmol of N-hydroxyphthalimide, and 3 ml of trifluoromethylbenzene were placed, and the resulting mixture was reacted at 60°C in an oxygen atmosphere (1 atm) for 5 hours with stirring. A gas chromatographic analysis of reaction products revealed that 1-nitroadamantane in a yield of 66% (on the basis of adamantane), 1-adamantanol in a yield of 4% (on the basis of adamantane), 2-adamantanone in a yield of 4%, 1,3-dinitroadamantane in a yield of 2% (on the basis of adamantane), 3-adamant-1-yl nitrate in a yield of 4% (on the basis of adamantane) were formed. The conversion rate from adamantane was 94%.

### EXAMPLE 9

In a flask, 1 mmol of 1,3-dimethyladamantane, 15 mmol of nitrogen dioxide, 0.1 mmol of N-hydroxyphthalimide, and 3 ml of trifluoromethylbenzene were placed, and the resulting mixture was reacted at 60°C in an oxygen atmosphere (1 atm) for 5 hours with stirring. A gas chromatographic analysis of reaction products revealed that 1,3-dimethyl-5-nitroadamantane was formed in a yield of 60% on the basis of 1,3-dimethyladamantane. The conversion rate from 1,3-dimethyladamantane was 82%.

### EXAMPLE 10

In a flask, 1 mmol of ethyl 1-adamantanecarboxylate, 15 mmol of nitrogen dioxide, 0.1 mmol of N-hydroxyphthalimide, and 3 ml of trifluoromethylbenzene were placed, and the resulting mixture was reacted at 60°C in an oxygen atmosphere (1 atm) for 15 hours with stirring. A gas chromatographic analysis of reaction products revealed that ethyl 3-nitro-1-adamantanecarboxylate was formed in a yield of 70% on the basis of ethyl 1-adamantanecarboxylate. The conversion rate from ethyl 1-adamantanecarboxylate was 99% or more.

### EXAMPLE 11

In a flask, 5 ml (38.4 mmol) of hexane, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitrohexanes and hexyl nitrate were formed in yields of 55% and 26%, respectively on the basis of nitrogen dioxide. The ratio of 2-nitrohexane to 3-nitrohexane [2-nitrohexane/3-nitrohexane] was 55/45.

### EXAMPLE 12

In a flask, 2.5 ml (23.2 mmol) of cyclohexane, 2.5 ml (19.2 mmol) of hexane, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitrocyclohexane, nitrohexanes, cyclohexyl nitrate, and hexyl nitrate were formed in yields of 39%, 19%, 5.6%, and 4%, respectively on the basis of nitrogen dioxide. The ratio of 2-nitrohexane to 3-nitrohexane [2-nitrohexane/3-nitrohexane] was 55/45.

### EXAMPLE 13

In a flask, 1 mmol of toluene, 15 mmol of nitrogen dioxide, and 3 ml of trifluoromethylbenzene were placed, and the resulting mixture was reacted at 60°C in an oxygen atmosphere (1 atm) for 5 hours with stirring. A gas chromatographic analysis of reaction products revealed that nitromethylbenzene (α-nitrotoluene) in a yield of 2% (on the basis of toluene), o-nitrotoluene in a yield of 51% (on the basis of toluene), m-nitrotoluene in a yield of 3%, and p-nitrotoluene in a yield of 34% were respectively formed. The conversion rate from toluene was 100%.

### COMPARATIVE EXAMPLE 1

In a flask, 1 mmol of toluene, 15 mmol of nitrogen dioxide, and 3 ml of trifluoromethylbenzene were placed, and the resulting mixture was reacted at 60°C in an argon atmosphere (1 atm) for 5 hours with stirring. A gas chromatographic analysis of reaction products revealed that nitromethylbenzene (α-nitrotoluene) in a yield of 3% (on the basis of toluene), o-nitrotoluene in a yield of 5% (on the basis of toluene), m-nitrotoluene in a yield of 1%, and p-nitrotoluene in a yield of 4% were respectively formed. The conversion rate from toluene was 37%.

### EXAMPLE 14

In a flask, 1 mmol of toluene, 0.1 mmol of N-hydroxyphthalimide, 15 mmol of nitrogen dioxide, and 3 ml of trifluoromethylbenzene were placed, and the resulting mixture was reacted at 60°C in an oxygen atmosphere (1 atm) for 5 hours with stirring. A gas chromatographic analysis of reaction products revealed that nitromethylbenzene (α-nitrotoluene) in a yield of 19% (on the basis of toluene), o-nitrotoluene in a yield of 36% (on the basis of toluene), m-nitrotoluene in a yield of 2%, and p-nitrotoluene in a yield of 18% were respectively formed. The conversion rate from toluene was 99%.

### EXAMPLE 15

In a flask, 1 mmol of toluene, 0.1 mmol of N-hydroxyphthalimide, 3 mmol of nitrogen dioxide, and 3 ml of trifluoromethylbenzene were placed, and the resulting mixture was reacted at 60°C in an oxygen atmosphere (1 atm) for 5 hours with stirring. A gas chromatographic analysis of reaction products revealed that nitromethylbenzene (α-nitrotoluene) in a yield of 30% (on the basis of toluene), o-nitrotoluene in a yield of 18% (on the basis of toluene), m-nitrotoluene in a yield of 1%, and p-nitrotoluene in a yield of 3% were respectively formed. The conversion rate from toluene was 99% or more.

### EXAMPLE 16

In a flask, 5 ml (46.9 mmol) of toluene, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitromethylbenzene (α-nitrotoluene) was formed in a yield of 52% on the basis of nitrogen dioxide and that 0.92 mmol of benzaldehyde was formed.

### EXAMPLE 17

In a flask, 5 ml (46.9 mmol) of toluene, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that nitromethylbenzene (α-nitrotoluene) was formed in a yield of 50% on the basis of nitrogen dioxide and that 0.44 mmol of benzaldehyde was formed.

### EXAMPLE 18

In a flask, 5 ml (40.9 mmol) of ethylbenzene, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that α-nitroethylbenzene and acetophenone oxime were formed in yields of 42% (on the basis of nitrogen dioxide) and 12%, respectively, and that 1.9 mmol of benzaldehyde and 1.2 mmol of α-hydroxyethylbenzene were formed.

### EXAMPLE 19

In a flask, 5 ml (40.9 mmol) of ethylbenzene and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that α-nitroethylbenzene was formed in a yield of 72% (on the basis of nitrogen dioxide) and that 1.4 mmol of benzaldehyde and 1.1 mmol of α-hydroxyethylbenzene were formed.

### EXAMPLE 20

In a flask, 5 ml (36.9 mmol) of p-t-butyltoluene, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that 1-t-butyl-4-nitromethylbenzene was formed in a yield of 55% on the basis of nitrogen dioxide, and that 1.0 mmol of p-t-butylbenzaldehyde and 0.44 mmol of p-t-butylbenzyl alcohol were formed.

### EXAMPLE 21

In a flask, 5 ml (36.9 mmol) of p-t-butyltoluene and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that 1-t-butyl-4-nitromethylbenzene was formed in a yield of 63% on the basis of nitrogen dioxide. Separately, 0.5 mmol of p-t-butylbenzaldehyde and 0.53 mmol of p-t-butylbenzyl alcohol were formed.

### EXAMPLE 22

In a flask, 3.2 mmol of durene, 0.6 mmol of N-hydroxyphthalimide, and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that 2,4,5-trimethyl-1-nitromethylbenzene, 2,4,5-trimethylbenzaldehyde, and 2,4,5-trimethylbenzoic acid were formed in yields of 30%, 9%, and 26%, respectively on the basis of durene.

### EXAMPLE 23

In a flask, 3.2 mmol of durene and 0.1 ml (3.24 mmol) of nitrogen dioxide were placed, and the resulting mixture was reacted at 70°C in an air atmosphere (1 atm) for 14 hours with stirring. A gas chromatographic analysis of reaction products revealed that 2,4,5-trimethyl-1-nitromethylbenzene and 2,4,5-trimethylbenzaldehyde were formed in yields of 28% and 8%, respectively on the basis of durene. Separately, a trace amount of 2,4,5-trimethylbenzoic acid was formed.

## Claims

1. A process for producing a nitro compound, said comprising the step of reacting nitrogen dioxide with an organic substrate in the presence of oxygen to yield a corresponding nitro compound.

2. A process for producing a nitro compound, said comprising the step of reacting nitrogen dioxide with an organic substrate in a molar ratio of said nitrogen dioxide to said organic substrate of less than 1 to yield a corresponding nitro compound.

3. A process according one of claims 1 and 2, wherein nitrogen dioxide is reacted with the organic substrate in the presence of an imide compound of the following formula (1): 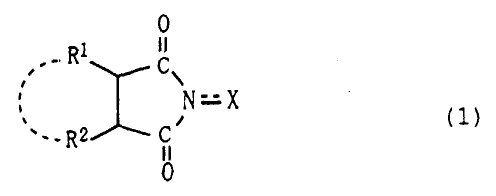 wherein each of R¹ and R² is, identical to or different from each other, a hydrogen atom, a halogen atom, an alkyl group, an aryl group, a cycloalkyl group, a hydroxyl group, an alkoxy group, a carboxyl group, an alkoxycarbonyl group, or an acyl group, where R¹ and R² may be combined to form a double bond, or an aromatic or non-aromatic ring; X is an oxygen atom or a hydroxyl group, where one or two N-substituted cyclic imido groups indicated in the formula (1) may be further formed on said R¹ or R², or on said double bond or aromatic or non-aromatic ring formed together by R¹ and R².

4. A process according to one of claims 1, 2 and 3, wherein said organic substrate is a compound selected from the group consisting of (a) aliphatic hydrocarbons, (b) alicyclic hydrocarbons, (c) non-aromatic heterocyclic compounds each having a carbon atom on a ring, said carbon atom being bonded to a hydrogen atom, (d) compounds each having a carbon-hydrogen bond at the adjacent position to an aromatic ring, and (e) compounds each having a carbon-hydrogen bond at the adjacent position to a carbonyl group.

5. A process for eliminating nitrogen dioxide, said process comprising the step of bringing a gas containing nitrogen dioxide into contact with an organic substrate in the presence of oxygen to eliminate the nitrogen dioxide through reaction.

6. A process for eliminating nitrogen dioxide, said process comprising the step of bringing a gas containing nitrogen dioxide into contact with an organic substrate in a proportion exceeding 1 mole relative to 1 mole of the nitrogen dioxide to eliminate the nitrogen dioxide through reaction.

7. A process according to one of claims 5 and 6, wherein the gas containing nitrogen dioxide is made into contact with the organic substrate in the presence of an imide compound of the following formula (1): 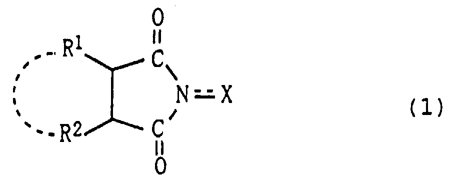 wherein each of R¹ and R² is, identical to or different from each other, a hydrogen atom, a halogen atom, an alkyl group, an aryl group, a cycloalkyl group, a hydroxyl group, an alkoxy group, a carboxyl group, an alkoxycarbonyl group, or an acyl group, where R¹ and R² may be combined to form a double bond, or an aromatic or non-aromatic ring; X is an oxygen atom or a hydroxyl group, where one or two N-substituted cyclic imido groups indicated in the formula (1) may be further formed on said R¹ or R², or on said double bond or aromatic or non-aromatic ring formed together by R¹ and R².

8. A process according to one of claims 5, 6 and 7, wherein said organic substrate is at least one compound selected from the group consisting of (a) aliphatic hydrocarbons, (b) alicyclic hydrocarbons, (c) non-aromatic heterocyclic compounds each having a carbon atom on a ring, said carbon atom being bonded to a hydrogen atom, (d) compounds each having a carbon-hydrogen bond at the adjacent position to an aromatic ring, and (e) compounds each having a carbon-hydrogen bond at the adjacent position to a carbonyl group.

9. A system for eliminating nitrogen dioxide, said system comprising eliminating means which brings a gas containing nitrogen dioxide into contact with an organic substrate in the presence of oxygen to eliminate the nitrogen dioxide through reaction.

10. A system for eliminating nitrogen dioxide, said system comprising eliminating means which brings a gas containing nitrogen dioxide into contact with an organic substrate in a proportion exceeding 1 mole relative to 1 mole of the nitrogen dioxide to eliminate the nitrogen dioxide through reaction.
